# EUROPÄISCHE PATENTSCHRIFT

(11) **EP 2 337 498 B1**
(45) Veröffentlichungstag und Bekanntmachung des Hinweises auf die Patenterteilung: **05.10.2016**
(21) Anmeldenummer: 09781220.0
(22) Anmeldetag: 29.07.2009
(51) Int. Cl.: A61B 6/14, A61B 5/107

(54) **VERFAHREN ZUR ERSTELLUNG EINER DENTALEN 3D-RÖNTGENAUFNAHME**
METHOD FOR PRODUCING A DENTAL 3D X-RAY IMAGE
PROCÉDÉ DE RÉALISATION D'UN ENREGISTREMENT RADIOGRAPHIQUE DENTAIRE EN 3 DIMENSIONS

(30) Priorität: 29.07.2008 DE 102008035412
(43) Veröffentlichungstag der Anmeldung: 29.06.2011
(73) Patentinhaber: Sirona Dental Systems GmbH, 64625 Bensheim (DE)
(72) Erfinder: ULRICI, Johannes, 64293 Darmstadt (DE); BONK, Roland, 67575 Eich (DE)
(74) Vertreter: Sommer, Peter
(86) Internationale Anmeldenummer: PCT/EP2009/059781
(87) Internationale Veröffentlichungsnummer: WO 2010/012754

(56) Entgegenhaltungen:
- EP-A- 1 815 794
- EP-A- 2 008 590
- DE-A1-102004 020 370
- JP-A- 2006 204 329
- JP-A- 2007 267 995
- US-A1- 2005 245 804
- US-A1- 2007 237 287

## Beschreibung

### Technisches Gebiet

Die Erfindung betrifft ein Verfahren zur Erstellung einer dentalen 3D-Röntgenaufnahme eines Objekts sowie ein Röntgengerät, wobei aus mehreren Projektionsaufnahmen während eines Umlaufs um das Objekt ein Volumen als 3D-Röntgenbild erstellbar ist.

### Stand der Technik

Bei derzeitigen sich auf dem Markt befindlichen Cone-Beam-Röntgengeräten zur Erstellung für dentale 3D-Röntgenaufnahmen im Kiefer- und Kopfbereich eines Patienten wird eine dreidimensionale Röntgenaufnahme eines Volumens erstellt. Das Volumen wird durch die Positionierung des Patienten bezüglich des Gerätes, durch die Auswahl der Umlaufbewegung von Röntgenstrahler und Röntgenbilddetektor um den Patienten herum sowie durch andere Größen wie Blendengröße, Strahlgeometrie usw. bezüglich seiner Lage und Größe bestimmt.

Für den Arzt besteht das Problem darin, die Lage und/oder die Größe des Volumens so auszuwählen, dass das aufgenommene Volumen mit dem medizinisch interessanten Bereich übereinstimmt. Dieses Problem ist um so größer, je kleiner das zu untersuchende Volumen ist. Daher stehen dem Bediener verschiedene Positionierhilfen zur Verfügung, denen meist mechanische oder optische Verfahren zugrunde liegen, beispielsweise die Anzeige der Positionierung durch ein Lichtvisier auf dem Patientenkopf.

Aus der DE 10 2004 020 370 A1 ist ein Röntgengerät bekannt, bei dem an einem Röntgenstrahler eine optische Kamera zur Erstellung einer optischen Aufnahme des mit dem Röntgenstrahler durchleuchteten Patientenkopfes vorgesehen ist. Dabei kann zu Beginn oder während des Umlaufs des Röntgenstrahlers um den Patientenkopf eine optische Aufnahme erzeugt werden, um für Diagnosezwecke die Zuordnung der Röntgenaufnahme zu der sichtbaren Oberfläche des Aufnahmeobjekts zu vereinfachen.

EP2130491 ist relevant unter Art. 54(3) EPÜ.

Dokument US2007237287 offenbart eine Röntgeneinrichtung zur Erstellung dentaler 3D-Röntgenaufnahmen, aufweisend eine Positioniervorrichtung zur Positionierung eines Patienten in ein Röntgengerät sowie ein System aus einem Röntgenstrahler (20) und einem Röntgenbilddetektor (20) als Teil des Röntgengeräts, die auf einer Umlaufbahn um den mit der Positioniervorrichtung positionierten Patienten herum bewegbar sind, wobei eine vor Erstellung der 3D-Röntgenaufnahme vorhandene bildlichen Darstellung zumindest eines Teil des Objekts vorhanden ist, dass Anzeigermittel für die bildliche Darstellung vorgesehen sind, dass weiterhin Eingabemittel zum Definieren eines auf dem Anzeigemittel dargestellten aufzunehmenden Volumens vorhanden sind. J

### Darstellung der Erfindung

Bei dem erfindungsgemäßen Verfahren zur Erstellung einer 3D-Röntgenaufnahme zumindest eines als Volumen ausgebildeten Teilbereichs eines Objekts mit einem Röntgengerät, umfassend die Positionierung des Objekts relativ zum Gerät, wobei aus mehreren Röntgen-Projektionsaufnahmen während eines zumindest teilweisen Umlaufs des Systems aus Röntgenstrahler und Röntgenbilddetektor um zumindest einen Teilbereich des Objekts ein Volumen als 3D-Röntgenbild erstellbar ist, wird vor der Erstellung der Röntgenaufnahme des Volumens mindestens ein Teil des Objekts in einer bildlichen Darstellung angezeigt, wobei die relative Lage der bildlichen Darstellung zu der aktuellen Geräte- und Patientenposition bekannt ist. Das von der Positionierung des Objekts bezüglich des Gerätes und von den Einstellungs- und/oder Steuerdaten abhängige aufzunehmende Volumen wird zumindest näherungsweise lagerichtig in die bildliche Darstellung eingeblendet.

Bei einer Veränderung der Lage und/oder Größe des Volumens in der bildlichen Darstellung werden die Einstellungs- und/oder Steuerdaten zur Erstellung der 3D-Röntgenaufnahme neu bestimmt oder angepasst.

Die Einstellungs- und/oder Steuerdaten legen beispielsweise die Lage der Drehachse des umlaufenden Systems aus Röntgenstrahler und Röntgenbilddetektor fest und gegebenenfalls Darstellung angezeigt, wobei aus mehreren optischen Aufnahmen ein optisches 3D-Bild oder eine dreidimensionale Kontur berechnet wird und wobei die relative Lage der bildlichen Darstellung zu der aktuellen Geräte- und Patientenposition bekannt ist. Das von der Positionierung des Objekts bezüglich des Gerätes und von den Einstellungs- und/oder Steuerdaten abhängige aufzunehmende Volumen wird zumindest näherungsweise lagerichtig in die dreidimensionale Kontur oder das optische 3D-Bild der bildlichen Darstellung eingeblendet.

Bei einer Veränderung der Lage und/oder Größe des Volumens in der bildlichen Darstellung werden die Einstellungs- und/oder Steuerdaten zur Erstellung der 3D-Röntgenaufnahme neu bestimmt oder angepasst.

Die Einstellungs- und/oder Steuerdaten legen beispielsweise die Lage der Drehachse des umlaufenden Systems aus Röntgenstrahler und Röntgenbilddetektor fest und gegebenenfalls deren Veränderung während des Umlaufs sowie weitere Parameter.

Die zumindest näherungsweise lagerichtige Einblendung kann beispielsweise dadurch erreicht werden, dass bei der Einblendung des aufzunehmenden Volumens in die bildliche Darstellung die relative Lage durch Vergleichen der jeweiligen Positionsdaten, also die Positionsdaten des aufzunehmenden Volumens und die Positionsdaten der bildlichen Darstellung, festgestellt wird und eine entsprechende Ausrichtung zueinander erfolgt. Es ist dabei ausreichend, dass das aufzunehmende Volumen als ein ungefährer Bereich angezeigt wird, wenn beispielsweise die Aufnahmewinkel bei den Aufnahmen unterschiedlich sind. Das zumindest schematisch eingeblendete Volumen gibt zwar die Lage und die Größe des als Volumen ausgebildeten Teilbereichs des Objekts wieder, hat jeerstellt worden sein oder als Standardaufnahme aufrufbar abgespeichert vorliegen. Die Verwendung einer bereits vorhandenen 3D-Aufnahme des Objekts zur Darstellung der Lage des kleineren Volumens innerhalb des Objekts hat den Vorteil, dass der Benutzer anhand bereits erzeugter Informationen und Strukturen, etwa der Verlauf von Nervenkanälen oder Zahnwurzeln, eine genauere Lagebestimmung und Auswahl der Größe des nun aufzunehmenden Volumens treffen kann.

Weiterhin können mehrere Aufnahmen des Objekts aus verschiedenen Richtungen erstellt und gleichzeitig angezeigt werden, wobei die Lage des Volumens in jeder dieser Aufnahmen zumindest näherungsweise lagerichtig dargestellt ist.

Besonders vorteilhaft ist es, wenn zusätzlich zu der Darstellung der Oberfläche und/oder Kontur des Objekts gleichzeitig eine gespeicherte 3D-Aufnahme angezeigt wird, wobei das zu erstellende Volumen sowohl in der Darstellung der Oberfläche und/oder Kontur des Objekts als auch in der gespeicherten 3D-Aufnahme angezeigt wird.

Durch diese Kombination der äußeren und inneren Merkmale des Objekts wird eine verbesserte Anordnung des Volumens erreicht.

Vorteilhafterweise können die Darstellung der Oberfläche und/oder der Kontur des Objekts und die vorhandene 3D-Aufnahme bezüglich ihrer Lage zueinander automatisch ausgerichtet werden und/oder Eingabemittel vorgesehen sein, um die Darstellung der Oberfläche und/oder der Kontur des Objekts und die vorhandene 3D-Aufnahme manuell bezüglich ihrer Lage zueinander auszurichten.

Vorteilhafterweise kann die bildliche Darstellung eine aus einer Mehrzahl von vorgegebenen typisierten anatomischen Strukturen auswählbare oder eine aus einer vorhandenen nahmen des Objekts aus verschiedenen Richtungen erstellt und gleichzeitig angezeigt werden und wobei die relative Lage der bildlichen Darstellung zu der aktuellen Geräte- und Patientenposition bekannt ist. Das von der Positionierung des Objekts bezüglich des Gerätes und von den Einstellungs- und/oder Steuerdaten abhängige aufzunehmende Volumen wird zumindest näherungsweise lagerichtig in jeder der mehreren optischen Aufnahmen der bildlichen Darstellung eingeblendet.

Bei einer Veränderung der Lage und/oder Größe des Volumens in der bildlichen Darstellung werden die Einstellungs- und/oder Steuerdaten zur Erstellung der 3D-Röntgenaufnahme neu bestimmt oder angepasst.

Hinsichtlich der beiden erfindungsgemäßen Verfahren ist es besonders vorteilhaft, wenn zusätzlich zu der Darstellung der Oberfläche und/oder Kontur des Objekts gleichzeitig eine gespeicherte 3D-Aufnahme angezeigt wird, wobei das aufzunehmende Volumen sowohl in der Darstellung der Oberfläche und/oder Kontur des Objekts als auch in der gespeicherten 3D-Aufnahme angezeigt wird.

Durch diese Kombination der äußeren und inneren Merkmale des Objekts wird eine verbesserte Anordnung des Volumens erreicht.

Vorteilhafterweise können die Darstellung der Oberfläche und/oder der Kontur des Objekts und die vorhandene 3D-Aufnahme bezüglich ihrer Lage zueinander automatisch ausgerichtet werden und/oder Eingabemittel vorhanden sein, mittels derer die Darstellung der Oberfläche und/oder der Kontur des Objekts und die vorhandene 3D-Aufnahme manuell bezüglich ihrer Lage zueinander ausgerichtet werden. aufweisend eine Positioniervorrichtung zur Positionierung des Patienten in einem Röntgengeräts sowie ein System aus einem Röntgenstrahler und einem Röntgenbilddetektor als Teil des Röntgengeräts, die auf einer Umlaufbahn um den in der Positioniervorrichtung positionierten Patienten herum bewegbar sind.

Weiterhin ist eine vor Erstellung der 3D-Röntgenaufnahme vorhandene bildlichen Darstellung zumindest eines Teil des Objekts vorhanden und sind Anzeigemittel für die bildliche Darstellung vorgesehen. Zur Veränderung eines auf dem Anzeigemittel dargestellten aufzunehmenden Volumens bezüglich der Lage und/oder Größe sind Eingabemittel vorhanden.

Dadurch lässt sich das in der bildlichen Darstellung schematisch dargestellte Volumen auf der Anzeigeeinheit verschieben. Als Anzeigemittel kommt sowohl die Bedienoberfläche des Röntgengeräts als auch eine vom Röntgengerät unabhängige Anzeigeeinheit in Betracht, die mit dem Röntgengerät lediglich datentechnisch verbunden ist und eine Software zur externen Steuerung des Röntgengeräts aufweisen kann.

Die vorhandene bildliche Darstellung kann vorzugsweise eine patientenbezogene oder standardisierte 3D-Aufnahme zumindest einer anatomischen Struktur und/oder mindestens ein Teil einer Oberfläche und/oder einer Kontur des Objekts sein. Die 3D-Aufnahme kann dabei beispielsweise eine optische Aufnahme, eine Röntgen- oder MRT-Aufnahme sein.

Weiterhin können Auswertemittel zur Festlegung der Einstellungs- und/oder Steuerdaten des Röntgengeräts, wie beispielsweise Bahnkurve und der Patientenposition, aus der Lage und/oder Größe des in der bildlichen Darstellung angezeigten Volumens vorgesehen sein. Das Gerät kann sich für die Erstellung der 3D-Röntgenaufnahme auf den mittels der Eingabemittel geänderten Auswahlbereich einstellen. Dies bedeutet, dass durch die Lage und die Größe des schematisch angezeigten Volumens die Einstellungs- und/oder Steuerdaten des Röntgengeräts zur Erstellung der 3D-Röntgenaufnahme bzw. der dafür erforderlichen Röntgen-Projektionsaufnahmen festlegt werden. Dazu kann auch die Patientenposition gehören.

Vorteilhafterweise kann an dem an dem System aus Röntgenstrahler und Röntgenbilddetektor eine optische Kamera angeordnet sein, die am Röntgenstrahler bzw. Röntgenbilddetektor oder seitlich des Röntgenstrahlers bzw. des Röntgenbilddetektors angebracht ist, wobei auch zwei oder mehr Kameras vorgesehen sein können. Die Lagebeziehung zwischen der mit der Kamera erstellten bildlichen Darstellung und dem aufzunehmenden Volumen ist dabei bekannt und mit der Kamera kann eine bildliche Darstellung des aufzunehmenden Objekts erzeugt werden.

### Kurzbeschreibung der Zeichnung

In der Zeichnung ist ein Ausführungsbeispiel der Erfindung wiedergegeben. Es zeigt:
- Fig. 1: eine dentale Röntgeneinrichtung zur Erstellung für 3D-Röntgenaufnahmen im Kiefer- und Kopfbereich;
- Fig. 2: die Röntgeneinrichtung aus Fig. 1 von oben;
- Fig. 3: eine schematische Darstellung der Anordnung des Objekts im Strahlengang;
- Fig. 4A,B: die Anzeige des aufzunehmenden Volumens in einer optischen Aufnahme;
- Fig. 5: die Anzeige des aufzunehmenden Volumens in einer optischen Aufnahme als Kontur;
- Fig. 6: die Anzeige des aufzunehmenden Volumens in einer optischen Aufnahme mit gleichzeitig eingeblendeter 3D-Röntgenaufnahme;
- Fig. 7: eine bildliche Darstellung des Volumens in einer Kontur mit gleichzeitig eingeblendeter anatomischer Struktur.

### Ausführungsbeispiel der Erfindung

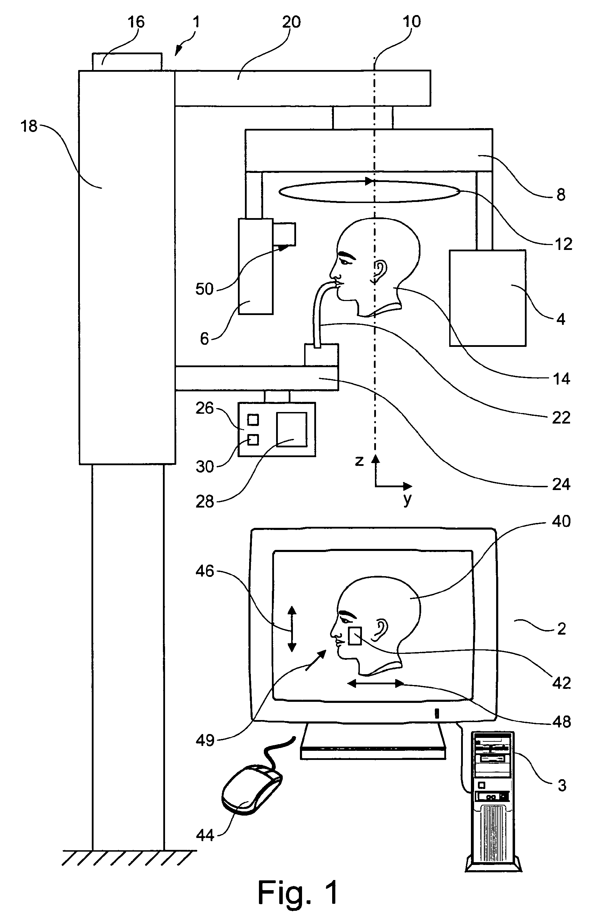

In Fig. 1 ist eine Röntgeneinrichtung zur Erstellung für 3D-Röntgenaufnahmen im Kiefer- und Kopfbereich dargestellt, welche ein Röntgengerät 1 und eine Anzeigeeinheit 2 umfasst, wobei die Anzeigeeinheit 2 Teil eines PC 3 sein kann, der mit der Röntgeneinrichtung 1 verbunden sein kann oder Teil dieser ist. Das Röntgengerät 1 weist ein aus einem Röntgenstrahler 4 und einem Röntgenbilddetektor 6 bestehendes System auf, wobei der Röntgenstrahler 4 und der Röntgenbilddetektor 6 über ein Träger 8 miteinander verbunden sind und wobei der Träger 8 um eine Drehachse 10 um einen Patientenkopf 14 als zu untersuchendes Objekt herumgeschwenkt werden kann. Dabei muss die Drehachse 10 während des Umlaufs nicht feststehend sein, sie kann zum Beispiel einer elliptischen Bewegung folgen.

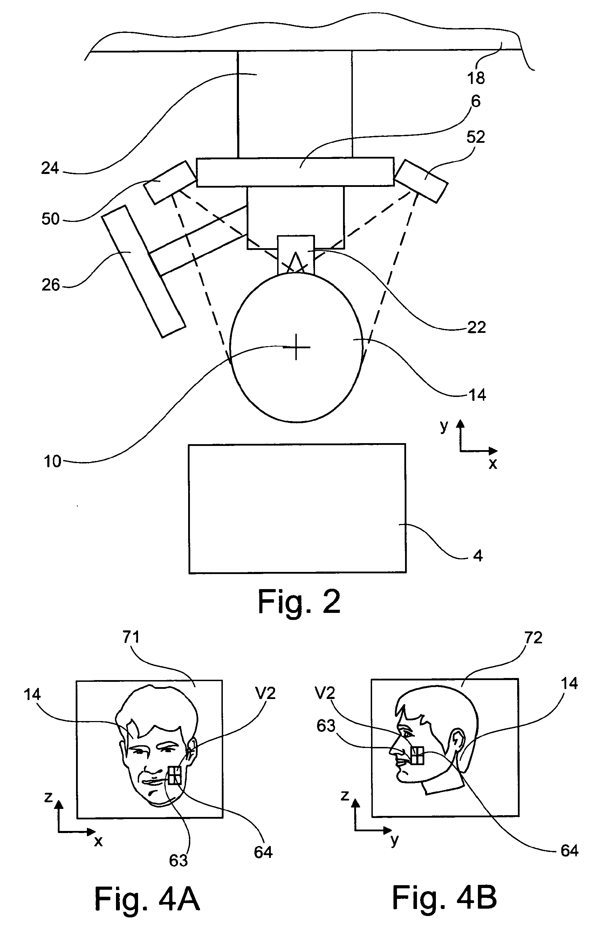

Der Träger 8 ist an einem entlang einer Säule 16 höhenverstellbar geführten Schlitten 18 mit einem Ausleger 20 drehbar befestigt. An dem höhenverstellbaren Schlitten 18 kann weiterhin ein Aufbiss 22 für die Positionierung des Patientenkopfs 14 vorgesehen sein. Der Aufbiss 22 kann an einem Ausleger 24 vorgesehen sein, der mit dem Schlitten 18 verbunden ist. An dem Ausleger 24 kann auch eine Bedienoberfläche 26 mit einer Anzeigeeinheit 28 sowie mit Bedienknöpfen 30 vorgesehen sein. Zur Erstellung einer bildlichen Darstellung des Objekts in Form von optischen Aufnahmen sind am Sensor 6 bzw. seitlich vom Sensor 6 Kameras vorgesehen, von denen eine Kamera 50 dargestellt ist. Dies wird später in Fig. 2 ausführlicher beschrieben.

Auf der Anzeigeeinheit 28 des Bedienteils 26 oder auf der externen Anzeige 2 wird das mit der Kamera 50 aufgenommene optische Bild 40 des Patientenkopfes 14 als bildliche Darstellung in Form einer optischen Seitenaufnahme dargestellt - hier in einer Aufnahmesituation der Kamera 50 in einem Winkel von 90° zu dem dargestellten Winkel. Das Bild 40 des Patientenkopfs, das auf der Anzeige 2 dargestellt ist, weist einen Bereich auf, in dem ein Volumen 42 angezeigt ist, das mit der Röntgeneinrichtung erzeugt werden soll. Dieses Volumen kann als Voreinstellung einer Standardposition folgen und angepasst werden, es kann aber auch aus mehreren Standardpositionen vorwählbar sein, wobei auch in diesem Fall eine nachträgliche Anpassung des Volumens an die tatsächlich durch den jeweiligen Patienten vorgegebene Situation möglich ist.

Die Lage des Volumens 42 in dem dargestellten Bild 40 des Kopfes kann dazu über die Bedienknöpfe 30 oder über ein Eingabemittel 44, etwa eine Computermaus, oder ein Lichtgriffel oder über einen berührungsempfindlichen Bildschirm verändert werden, angedeutet durch die Pfeile 46, 48. Weiterhin ist es möglich, die Größe des Volumens 42 zu verändern, etwa über ein vom Eingabemittel 44 bewegten Eingabepfeil 49.

Die Übertragung der Lage und Größe des dargestellten Volumens 42 zur Festlegung der Einstellungs- und/oder Steuerdaten der Röntgeneinrichtung für die Erstellung der 3D-Röntgenaufnahme des Volumens 42 kann über Auswertemittel erfolgen, die Teil des PC 3 sind. Mit dem PC 3 kann auch aus den unterschiedlichen Projektionsaufnahmen die 3D-Röntgenaufnahme des Volumens 42 erzeugt werden.

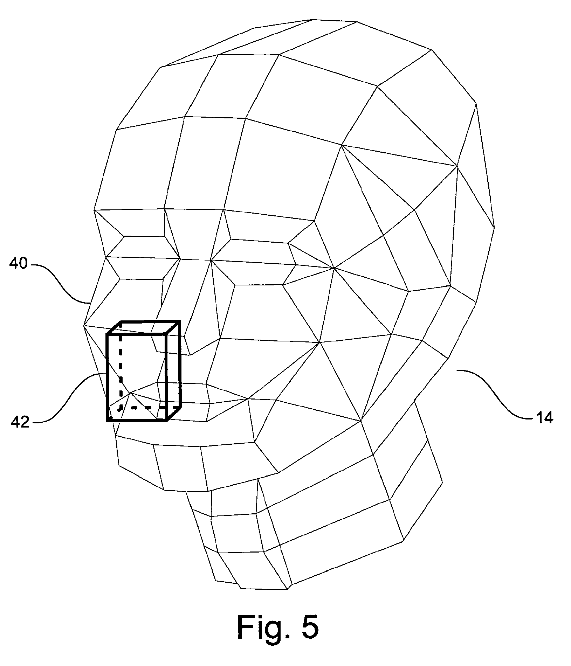

Die Aufnahme 40 des Patientenkopfs oder eines Teilbereichs davon kann auch ein optisches 3D-Bild sein, das aus mehreren optischen Aufnahmen, die aus unterschiedlichen Richtungen erstellt worden sind, gebildet wurde und beispielsweise als Kontur 40 dargestellt wird (Fig.5). Theoretisch reichen jedoch bereits zwei Projektionen aus unterschiedlichen Richtungen aus, um die Lage eines kleineren Volumens 42 eindeutig zu bestimmen. Anstelle einer 3D-Aufnahme können daher eine seitliche Aufnahme des Patientenkopfs und eine Frontalaufnahme dargestellt sein, auf denen die jeweilige Lage des Volumens angegeben ist, siehe hierzu Fig. 4A,B.

Die Aufnahme 40 kann aber auch aus einer Vermessung des Patientenkopfs innerhalb der Röntgeneinrichtung gewonnen worden sein, wobei Messdaten auch zunächst als Einzelaufnahmen aus einer bestimmten Richtung erstellt werden. Derartige Scan-Verfahren sind im Stand der Technik bekannt. Diese Messdaten können gegebenenfalls durch weitere Messaufnahmen aus unterschiedlichen Richtungen ergänzt werden.

Anstelle optischer Aufnahmen kann das Objekt z.B. mechanisch oder optisch vermessen werden, wobei einige Messpunkte ausreichen können, um dann aus den Messpunkten eine Kontur schematisch zu berechnen, siehe Fig. 5.

In Fig. 2 ist eine Prinzipskizze für die Erstellung von optischen Aufnahmen gezeigt, ausgehend von dem mittels des Aufbisses 22 über den Ausleger 24 und den Schlitten 18 in der Röntgeneinrichtung bezüglich seiner Lage festgelegten Patientenkopf 14 als zu untersuchendes Objekt. Zu beiden Seiten des Sensors 6 sind optische Kameras 50, 52 vorgesehen, die aus unterschiedlichen Richtungen auf den Patientenkopf 14 ausgerichtet sind. Aus diesen beiden Aufnahmen mit bekannten Lagebeziehungen zum aufzunehmen Objekt lässt sich der Patientenkopf 14 gleichzeitig aus zwei Richtungen darstellen.

Sollten zwei Aufnahmen nicht ausreichen, kann der Sensor 6 mit den beiden Kameras 50, 52 um die Drehachse 10 gedreht werden, wodurch weitere Aufnahmen aus unterschiedlicher Richtung bereitgestellt werden können.

Auf diese Art und Weise kann auch eine optische 3D-Vermessung durchgeführt werden, so dass anstelle einer optischen Aufnahme ein optischer 3D-Messdatensatz des Patientenkopfes vorliegt.

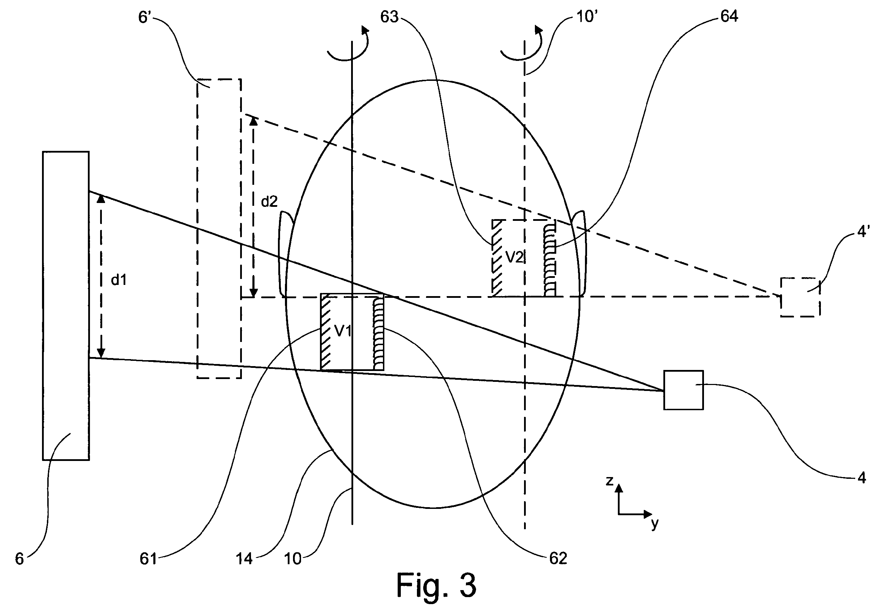

In Fig. 3 sind zwei im frontal gezeigten Patientenkopf 14 an unterschiedlichen Positionen angeordnete Volumen V1, V2 dargestellt. Die Position und die Größe des Volumens V1, V2 hat Auswirkungen auf die Positionierung des Patienten 14 bezüglich des mit Strahler 4, 4' und Sensor 6, 6' versehenen Geräts, auf die Lage der Bahnkurve des Umlaufs des Strahlers 4, 4' und Sensors 6, 6'um die Drehachse 10, 10', auf die Blendeneinstellung zur Begrenzung des Strahlkegels und gegebenenfalls auf weitere Parameter.

Dabei kann auch die Größe d1, d2 des belichteten Bereichs des Sensors 6, 6' von der Lage und Größe des Volumens V1, V2 abhängen.

Die jeweils auf dem Patientenkopf 14 an dessen Oberfläche abgedeckten Grenzen 61, 62 für das Volumen V1 bzw. Grenzen 63, 64 für das Volumen V2 werden in der in den Fig. 4A, 4B beispielsweise wiedergegebenen optischen Aufnahmen für das Volumen V2 dargestellt. Dabei kann ein kegelförmiger Strahl berücksichtigt werden, wobei eine größen- und lagerichtige Anzeige des Volumens angestrebt ist.

Fig. 4A ist dabei eine optische Aufnahme 71 in einer z-x-Ebene als frontale Vollaufnahme des Patientenkopfs 14, Fig. 4B ist eine optische Aufnahme 72 in einer z-y-Ebene als Seitenaufnahme des Patientenkopfs 14.

Eine Veränderung der Lage der Grenzen 63, 64 für das Volumen V2 verändert auch das Volumen V2 und die für die Erstellung des Volumens V2 erforderlichen Geräteparameter.

In Fig. 5 wird die Anzeige des aufzunehmenden Volumens 42 in einer optischen Aufnahme 40 des Patientenkopfs 14 als Kontur gezeigt. Dabei kann das aufzunehmende Volumen 42 perspektivisch dargestellt sein.

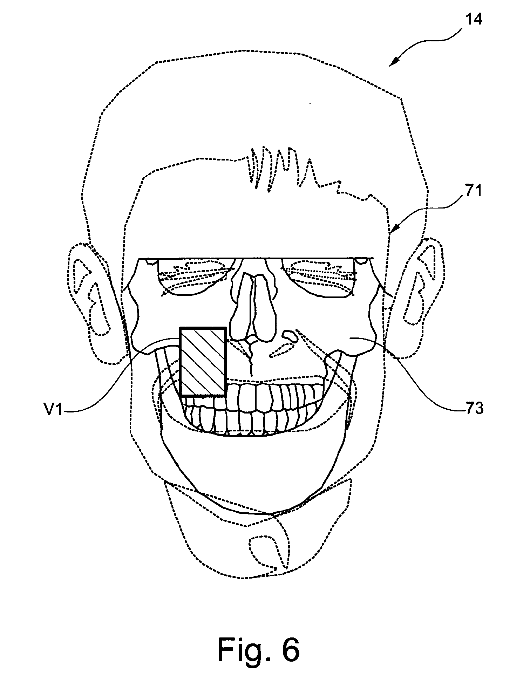

In Fig. 6 ist zusätzlich zu der bildlichen Darstellung der Aufnahme 71 der Oberfläche des Objekts aus Fig. 4A eine gespeicherte 3D-Aufnahme 73 angezeigt, wobei anstelle der Vollaufnahme des Patientenkopfes auch die Darstellung des unteren Gesichtsbereichs als Teilaufnahme ausreichend sein kann. Über Eingabemittel können die Aufnahme 71 des Objekts und die 3D-Röntgenaufnahme 73 bezüglich ihrer Lage zueinander ausgerichtet werden, wobei das zu erstellende Volumen V1 sowohl in der optischen Aufnahme 71 der Schädelkontur als auch in der 3D-Röntgenaufnahme 73 angezeigt wird und bezüglich der Lage und Größe veränderbar ist.

Die räumliche Auflösung des Volumens V1 ist nach der Erstellungen der 3D-Röntgenaufnahme dieses Volumens 42 gegenüber der Auflösung der gespeicherten 3D-Röntgenaufnahme 73 höher und erlaubt daher eine genauere Auswertung.

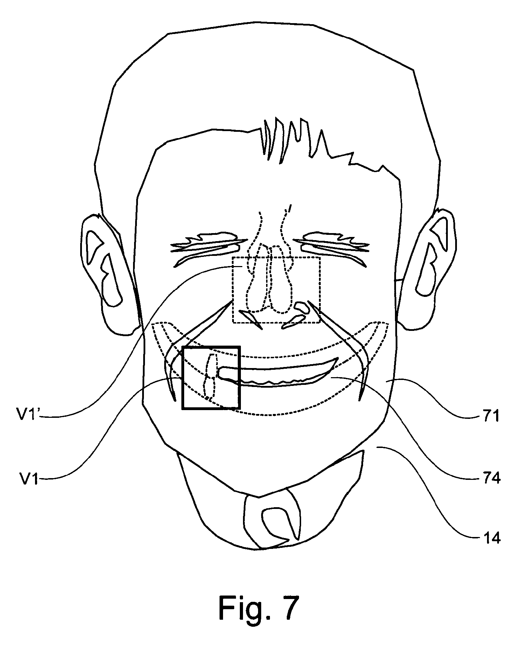

Fig. 7 betrifft eine bildliche Darstellung in Form eines Kieferkammbogens 74 als Beispiel einer anatomischen Struktur innerhalb der optischen Objektdarstellung 71, wodurch das Positionieren einfacher wird. Der Kieferkammbogen 74 kann entweder aus einer vorherigen Aufnahme, etwa auch aus einer Panoramaschichtaufnahme des Patienten generiert werden. Auch eine schematische Darstellung der anatomischem Struktur ist möglich, wobei der Zahnarzt selbst die Form des Kiefers beurteilt und gegebenenfalls aus verschiedenen vorhandenen Schemata ein geeignetes aussucht. Ebenfalls möglich ist die Ableitung eines zu erwartenden Kieferkammbogenschemas aus der Kopfkontur.

Falls für die Positionierung des Volumens V1 eine Einschränkung auf mindestens einen Teilbereich der anatomischen Struktur 74 vorliegt, kann die Positionierung zuverlässiger erfolgen, da diese Struktur dann erwartungsgemäß auch getroffen wird. Hierbei können Grenzwerte für die Mindestüberdeckung zweckmäßig sein, etwas mindestens 25%-50%, abhängig vom Aufnahmetyp. Eine Anordnung des Volumens V1' außerhalb der anatomischen Struktur 74 wäre damit ausgeschlossen. Dieses Erfordernis könnte vom Aufnahmetyp abhängen, also von einer Vorauswahl der Aufnahmeart, etwa Zahnwurzel, Kiefergelenk oder ähnliches

Grundsätzlich gilt, dass die zumindest näherungsweise lagerichtige Einblendung des aufzunehmenden Volumens in eine bildliche Darstellung des Objekts, etwa als optische Aufnahme oder in eine bereits vorhandene 3D-Aufnahme eine Art Lage-Registrierung erfordert, welche die Lage der bildlichen Darstellung bezüglich der aktuellen Geräte- und Patientenposition definiert. Beispielsweise können hierfür die Daten aus der Position der Patientenfixierung bei der Erstellung der bildlichen Darstellung erfasst und gespeichert worden sein, sodass ein Vergleich mit der aktuellen Position möglich ist.

Allgemeinen formuliert bedeutet dies, dass ein Vergleich der relativen Position des Gerätes zum Patienten bei der vorherigen Röntgenaufnahme mit der aktuellen Geräte- und Patientenposition erfolgt, um die Position des aufzunehmenden Volumens in der bildlichen Darstellung korrekt darzustellen. Wenn die optische Aufnahme nicht unmittelbar vor der Röntgenaufnahme des Volumens erfolgt, ist dies auch bei den optischen Aufnahmen nach Fig. 2 erforderlich.

## Patentansprüche

1. Verfahren zur Erstellung einer dentalen 3D-Röntgenaufnahme zumindest eines als ein Volumen (42, V1, V2) ausgebildeten Teilbereichs eines Objekts (14) mit einem Röntgengerät (1), umfassend die Positionierung des Objekts (14) relativ zum Gerät (1), wobei aus mehreren Röntgen-Projektionsaufnahmen während eines zumindest teilweisen Umlaufs des Systems aus einem Röntgenstrahler (4, 4') und einem Röntgenbilddetektor (6, 6') um zumindest einen Teilbereich des Objekts (14) das Volumen (42, V1, V2) als die 3D-Röntgenaufnahme erstellt wird, **dadurch gekennzeichnet,**
- **dass** vor der Erstellung der 3D-Röntgenaufnahme des Volumens (42,V1,V2) mindestens ein Teil des Objekts (14) in einer mindestens einen Teil einer Oberfläche und/oder einer Kontur des Objekts (14) aufweisenden bildlichen Darstellung (40, 71, 72, 73) angezeigt wird, wobei aus mehreren optischen Aufnahmen (40) ein optisches 3D-Bild oder eine dreidimensionale Kontur berechnet wird und wobei die relative Lage der bildlichen Darstellung (40, 71, 72, 73, 74) zu der aktuellen Geräte- und Patientenposition bekannt ist,
- **dass** das von der Positionierung des Objekts (14) bezüglich des Gerätes und von der Wahl der Einstellungs- und/oder Steuerdaten abhängige aufzunehmende Volumen (42, V1, V2) in die dreidimensionale Kontur oder das optische 3D-Bild der bildlichen Darstellung (40, 71, 72, 73, 74) zumindest näherungsweise lagerichtig eingeblendet wird,
- **dass** bei einer Veränderung der Lage und/oder Größe des aufzunehmenden Volumens (42, V1, V2) in der bildlichen Darstellung (40, 71, 72) die Einstellungs- und/oder Steuerdaten zur Erstellung der 3D-Röntgenaufnahme bestimmt werden,
- **dass** die Lage und/oder Größe des in der bildlichen Darstellung (40, 71, 72, 73, 74) wiedergegeben Volumens (42, V1, V2) vor der Erstellung der Röntgenaufnahme verändert wird, wobei die relative Lage des Patienten zum Gerät und/oder die zur Erstellung des Volumens (42, V1, V2) notwendigen Einstellungs- und/oder Steuerdaten an die geänderte Lage und/oder Größe des Volumens (42, V1, V2) angepasst wird,
- **dass** das auf einer Anzeige (2) dargestellte Volumen (42, V1, V2) mittels eines Eingabegeräts (44) auf der Anzeige (2) verschoben wird und dass die Positionsdaten und/oder Größe des angezeigten Volumens (42, V1, V2) an das Röntgengerät (1) weitergegeben werden und
- **dass** das auf einer Anzeige (2) dargestellte, mittels des Eingabegeräts (44) auf der Anzeige (2) verschiebbar Volumen (42, V1, V2) nur unter Beibehaltung einer zumindest teilweisen Überdeckung mit der dargestellten anatomischen Struktur (74) verschoben wird.

2. Verfahren zur Erstellung einer dentalen 3D-Röntgcnaufnahme zumindest eines als ein Volumen (42, V1, V2) ausgebildeten Teilbereichs eines Objekts (14) mit einem Röntgengerät (1), umfassend die Positionierung des Objekts (14) relativ zum Gerät (1), wobei aus mehreren Röntgen-Projektionsaufnahmen während eines zumindest teilweisen Umlaufs des Systems aus einem Röntgenstrahler (4, 4') und einem Röntgenbilddetektor (6, 6') um zumindest einen Teilbereich des Objekts (14) das Volumen (42, V1, V2) als die 3D-Röntgenaufnahme erstellt wird, **dadurch gekennzeichnet,**
- **dass** vor der Erstellung der 3D-Röntgenaufnahme des Volumens (42,V1,V2) mindestens ein Teil des Objekts (14) in einer mindestens einen Teil eine Oberfläche und/oder einer Kontur des Objekts (14) aufweisenden bildlichen Darstellung (40, 71, 72, 73) angezeigt wird, wobei mehrere optische Aufnahmen (71, 72) des Objekts (14) aus verschiedenen Richtungen erstellt und angezeigt werden und wobei die relative Lage der bildlichen Darstellung (40, 71, 72, 73, 74) zu der aktuellen Geräte- und Patientenposition bekannt ist,
- **dass** das von der Positionierung des Objekts (14) bezüglich des Gerätes und von der Wahl der Einstellungs- und/oder Steuerdaten abhängige aufzunehmende Volumen (42, V1, V2) in jeder der mehreren optischen Aufnahmen (71, 72) der bildlichen Darstellung (40, 71, 72, 73, 74) zumindest näherungsweise lagerichtig eingeblendet wird,
- **dass** bei einer Veränderung der Lage und/oder Größe des aufzunehmenden Volumens (42, V1, V2) in der bildlichen Darstellung (40, 71, 72) die Einstellungs- und/oder Steuerdaten zur Erstellung der 3D-Röntgenaufnahme bestimmt werden,
- **dass** die Lage und/oder Größe des in der bildlichen Darstellung (40, 71, 72, 73, 74) wiedergegeben Volumens (42, V1, V2) vor der Erstellung der Röntgenaufnahme verändert wird, wobei die relative Lage des Patienten zum Gerät und/oder die zur Erstellung des Volumens (42, V1, V2) notwendigen Einstellungs- und/oder Steuerdaten an die geänderte Lage und/oder Größe des Volumens (42, V1, V2) angepasst wird,
- **dass** das auf einer Anzeige (2) dargestellte Volumen (42, V1, V2) mittels eines Eingabegeräts (44) auf der Anzeige (2) verschoben wird und dass die Positionsdaten und/oder Größe des angezeigten Volumens (42, V1, V2) an das Röntgengerät (1) weitergegeben werden und
- **dass** das auf einer Anzeige (2) dargestellte, mittels des Eingabegeräts (44) auf der Anzeige (2) verschiebbar Volumen (42, V1, V2) nur unter Beibehaltung einer zumindest teilweisen Überdeckung mit der dargestellten anatomischen Struktur (74) verschoben wird.

3. Verfahren nach einem der Ansprüche 1 oder 2, **dadurch gekennzeichnet, dass** zusätzlich zu der Darstellung der Oberfläche und/oder Kontur des Objekts (40, 71, 72) gleichzeitig eine gespeicherte 3D-Aufnahme (73) angezeigt wird, wobei das aufzunehmende Volumen (42, V1, V2) sowohl in der Darstellung der Oberfläche und/oder Kontur des Objekts (40, 71, 72) als auch in der 3D-Aufnahme (73) angezeigt wird.

4. Verfahren nach Anspruch 3, **dadurch gekennzeichnet, dass** die Darstellung der Oberfläche und/oder der Kontur des Objekts (40, 71, 72) und die vorhandene 3D-Aufnahme (73) bezüglich ihrer Lage zueinander automatisch ausgerichtet werden und/oder dass Eingabemittel (44) vorhanden sind, mittels derer die Darstellung der Oberfläche und/oder Kontur des Objekts (40, 71, 72) und die vorhandene 3D-Aufnahme (73) bezüglich ihrer Lage zueinander ausgerichtet werden.

5. Verfahren nach einem der Ansprüche 1 bis 4, **dadurch gekennzeichnet, dass** zusätzlich innerhalb der bildlichen Darstellung eine aus einer Mehrzahl von vorgegebenen typisierten anatomischen Strukturen (74), wie beispielsweise Kieferkammbögen (74), auswählbare oder eine aus einer vorhandenen sonstigen Darstellung erzeugbare anatomische Struktur angezeigt wird.

## Claims

1. A method for preparing a 3-D dental x-ray image of at least one section of an object (14) configured as a volume (42, V1, V2) using an x-ray machine (1), comprising the positioning of the object (14) relative to the machine (1), wherein the volume (42, V1, V2) is created as the 3-D x-ray image from a plurality of x-ray projection images during an at least partial revolution of the system consisting of an x-ray tube (4, 4') and an x-ray image detector (6, 6') around at least a section of the object (14), **characterized in that**,
- before creating the 3-D x-ray image of the volume (42, V1, V2), at least part of the object (14) is displayed in a graphic representation (40, 71, 72, 73) having at least part of a surface and/or a contour of the object (14), wherein a visual 3-D image or a three-dimensional contour is calculated from a plurality of visual images (40), and wherein the relative position of the graphic representation (40, 71, 72, 73, 74) to the current machine and patient position is known,
- the volume (42, V1, V2) that is to be imaged and depends on the position of the object (14) relative to the machine, and on the selection of the adjustment and/or control data, is shown at least approximately in the correct position in the three-dimensional contour or the visual 3-D image of the graphic representation (40, 71, 72, 73, 74),
- when the position and/or size of the volume (42, V1, V2) to be imaged changes in the graphic representation (40, 71, 72), the adjustment and/or control data for creating the 3-D x-ray image are determined,
- the position and/or size of the volume (42, V1, V2) reproduced in the graphic representation (40, 71, 72, 73, 74) is changed before creating the x-ray image, wherein the relative position of the patient to the machine and/or the adjustment and/or control data needed to create the volume (42, V1, V2) is adapted to the changed position and/or size of the volume (42, V1, V2),
- the volume (42, V1, V2) depicted on a display (2) is moved on the display (2) by means of an input device (44), and the position data and/or the size of the displayed volume (42, V1, V2) are transmitted to the x-ray machine (1), and
- the volume (42, V1, V2) that is depicted on a display (2) and can be moved on the display (2) by means of the input device (44) is only moved while retaining at least a partial overlap with the depicted anatomical structure (74).

2. A method for preparing a 3-D dental x-ray image of at least one section of an object (14) configured as a volume (42, V1, V2) using an x-ray machine (1), comprising the positioning of the object (14) relative to the machine (1), wherein the volume (42, V1, V2) is created as the 3-D x-ray image from a plurality of x-ray projection images during an at least partial revolution of the system consisting of an x-ray tube (4, 4') and an x-ray image detector (6, 6') around at least a section of the object (14), **characterized in that**,
- before creating the 3-D x-ray image of the volume (42, V1, V2), at least part of the object (14) is displayed in a graphic representation (40, 71, 72, 73) having at least part of a surface and/or a contour of the object (14), wherein a plurality of visual images (71, 72) of the object (14) is created and displayed from different directions, and wherein the relative position of the graphic representation (40, 71, 72, 73, 74) to the current machine and patient position is known,
- the volume (42, V1, V2) that is to be imaged and depends on the position of the object (14) relative to the machine, and on the selection of the adjustment and/or control data, is shown at least approximately in the correct position in each of the plurality of visual images (71, 72) of the graphic representation (40, 71, 72, 73, 74),
- when the position and/or size of the volume (42, V1, V2) to be imaged changes in the graphic representation (40, 71, 72), the adjustment and/or control data for creating the 3-D x-ray image are determined,
- the position and/or size of the volume (42, V1, V2) reproduced in the graphic representation (40, 71, 72, 73, 74) is changed before creating the x-ray image, wherein the relative position of the patient to the machine and/or the adjustment and/or control data needed to create the volume (42, V1, V2) is adapted to the changed position and/or size of the volume (42, V1, V2),
- the volume (42, V1, V2) depicted on a display (2) is moved on the display (2) by means of an input device (44), and the position data and/or the size of the displayed volume (42, V1, V2) are transmitted to the x-ray machine (1), and
- the volume (42, V1, V2) that is depicted on a display (2) and can be moved on the display (2) by means of the input device (44) is only moved while retaining at least a partial overlap with the depicted anatomical structure (74).

3. A method according to one of claims 1 or 2, **characterized in that** a saved 3-D image (73) is simultaneously displayed in addition to the depiction of the surface and/or the contour of the object (40, 71, 72), wherein the volume (42, V1, V2) to be imaged is displayed both in the depiction of the surface and/or contour of the object (40, 71, 72) and in the 3-D image (73).

4. The method according to claim 3, **characterized in that** the depiction of the surface and/or the contour of the object (40, 71, 72) and the available 3-D image (73) are automatically aligned with regard to their position relative to each other, and/or input means (44) are available by means of which the depiction of the surface and/or contour of the object (40, 71, 72) and the available 3-D image (73) are aligned with regard to their position relative to each other.

5. The method according to one of claims 1 to 4, **characterized in that** an anatomical structure is displayed which can be selected from a plurality of predetermined, standardized anatomical structures (74), such as for example alveolar ridges (74), or can be generated from another available depiction.

## Revendications

1. Procédé de réalisation d'un enregistrement radiographique dentaire en trois dimensions d'au moins une zone partielle, formée comme volume (42, V1, V2), d'un objet (14) avec un appareil de radiographie (1), comprenant le positionnement de l'objet (14) par rapport à l'appareil (1), où, le volume (42, V1, V2) est réalisé, caractérisé sous forme d'enregistrement radiographique en 3D à partir de plusieurs enregistrements radiographiques en projection pendant une rotation au moins partielle du système, à partir d'un émetteur de rayons X (4, 4') et d'un détecteur d'image radiographique (6, 6') autour d'au moins une zone partielle de l'objet (14),
- en ce que, avant la réalisation de l'enregistrement radiographique en 3D du volume (42, V1, V2), au moins une partie de l'objet (14) est affichée sous forme imagée (40, 71, 72, 73) présentant au moins une partie d'une surface et/ou d'un contour de l'objet (14), où, une image en 3D optique ou un contour en trois dimensions est calculé(e) à partir de plusieurs enregistrements optiques (40) et où la position relative de la représentation imagée (40, 71, 72, 73, 74) par rapport à la position effective de l'appareil et du patient est connue,
- en ce que le volume (42, V1, V2) qui doit être enregistré en fonction du positionnement de l'objet (14) par rapport à l'appareil et de la sélection des données de réglage et/ou de commande est affiché dans le contour en trois dimensions ou dans l'image en 3D optique de la représentation imagée (40, 71, 72, 73, 74) dans une position à peu près correcte,
- en ce que, en cas de modification de la position et/ou de la taille du volume (42, V1, V2) enregistrée dans la représentation imagée (40, 71, 72), des données de réglage et/ou de commande sont déterminées pour établir les enregistrements radiographiques en 3D,
- en ce que la position et/ou la taille du volume (42, V1, V2) reproduit dans la représentation imagée (40, 71, 72, 73, 74) sont modifiées avant la réalisation de l'enregistrement radiographique, où la position relative du patient par rapport à l'appareil et/ou les données de réglage et/ou de commande nécessaires pour la réalisation du volume (42, V1, V2) sont adaptées à la position et/ou à la taille modifiée(s) du volume (42, V1, V2),
- en ce que le volume (42, V1, V2) représenté sur un affichage (2) est déplacé sur l'affichage (2) au moyen d'une unité d'entrée (44) et en ce que les données de position et/ou la taille du volume (42, V1, V2) affichées sont transmises à l'appareil de radiographie (1) et
- en ce que le volume (42, V1, V2) représenté sur un affichage (2), pouvant être déplacé au moins d'une unité d'entrée (44) sur l'affichage (2) est décalé uniquement en tenant compte d'un recouvrement au moins partiel avec la structure anatomique (74) représentée.

2. Procédé de réalisation d'un enregistrement radiographique dentaire en trois dimensions d'au moins une zone partielle, formée comme volume (42, V1, V2), d'un objet (14) avec un appareil de radiographie (1), comprenant le positionnement de l'objet (14) par rapport à l'appareil (1), où, le volume (42, V1, V2) est réalisé caractérisé à partir de plusieurs enregistrements radiographiques en projection pendant une rotation au moins partielle du système à partir d'un émetteur de rayons X (4, 4') et un détecteur d'image radiographique (6, 6') autour d'au moins une zone partielle de l'objet (14), sous forme d'enregistrement radiographique en 3D,
- en ce que, avant la réalisation de l'enregistrement radiographique en 3D du volume (42, V1, V2), au moins une partie de l'objet (14) est affichée dans une représentation imagée (40, 71, 72, 73) présentant au moins une partie d'une surface et/ou d'un contour de l'objet (14), où plusieurs enregistrements optiques (71, 72) de l'objet (14) sont réalisés depuis différentes directions puis affichés et où la position relative de la représentation imagée (40, 71, 72, 73, 74) par rapport à la position effective de l'appareil et du patient est connue,
- en ce que le volume (42, V1, V2) qui doit être enregistré en fonction du positionnement de l'objet (14) par rapport à l'appareil et de la sélection des données de réglage et/ou de commande est introduit dans chacun des différents enregistrements optiques (71, 72) de la représentation imagée (40, 71, 72, 73, 74) dans une position au moins approximativement correcte,
- en ce que, en cas de modification de la position et/ou de la taille du volume (42, V1, V2) enregistrée dans la représentation imagée (40, 71, 72), des données de réglage et/ou de commande sont déterminées pour établir les enregistrements radiographiques en 3D,
- en ce que la position et/ou la taille du volume (42, V1, V2) reproduit dans la représentation imagée (40, 71, 72, 73, 74) sont modifiées avant la réalisation de l'enregistrement radiographique, où la position relative du patient par rapport à l'appareil et/ou les données de réglage et/ou de commande nécessaires pour la réalisation du volume (42, V1, V2) sont adaptées à la position et/ou la taille modifiée(s) du volume (42, V1, V2),
- en ce que le volume (42, V1, V2) représenté sur un affichage (2) est déplacé sur l'affichage (2) au moyen d'un appareil d'entrée (44) et en ce que les données de position et/ou la taille du volume (42, V1, V2) affiché sont transmises à l'appareil de radiographie (1) et
- en ce que le volume (42, V1, V2) représenté sur un affichage (2), pouvant être déplacé au moins d'une unité d'entrée (44) sur l'affichage (2) est décalé uniquement en tenant compte d'un recouvrement au moins partiel avec la structure anatomique (74) représentée.

3. Procédé selon l'une des revendications 1 ou 2, **caractérisé en ce que**, outre la représentation de la surface et/ou du contour de l'objet (40, 71, 72), un enregistrement en 3D mémorisé (73) s'affiche simultanément, dans lequel le volume (42, V1, V2) à enregistrer est affiché à la fois dans la représentation de la surface et/ou du contour de l'objet (40, 71, 72) et dans l'enregistrement en 3D (73).

4. Procédé selon la revendication 3, **caractérisé en ce que** les positions de la représentation de la surface et/ou du contour de l'objet (40, 71, 72) et l'enregistrement en 3D disponible (73) sont alignés automatiquement les uns par rapport aux autres et/ou **en ce que** des moyens d'entrée (44) sont disponibles, permettant d'aligner ces positions de la représentation de la surface et/ou du contour de l'objet (40, 71, 72) et l'enregistrement en 3D disponible (73) les uns par rapport aux autres.

5. Procédé selon l'une des revendications 1 à 4, **caractérisé en ce que**, en outre, à l'intérieur de la représentation imagée, une structure parmi une pluralité de structures anatomiques (74) standard prédéfinies, par exemple les voûtes de la crête maxillaire (74), ou une structure anatomique pouvant être sélectionnée ou réalisée à partir d'une autre représentation disponible, est affichée.
